# EUROPEAN PATENT APPLICATION

(11) **EP 4 640 224 A1**
(43) Date of publication of application: **29.10.2025**
(21) Application number: 23907910.6
(22) Date of filing: 15.12.2023
(51) Int. Cl.: A61K 31/7034, A61K 31/7048, A61K 31/4184, A61K 45/06, A61P 1/16, A61P 3/00

(54) **PHARMACEUTICAL COMPOSITION CONTAINING SODIUM-GLUCOSE TRANSPORTER-2 INHIBITOR AND ANGIOTENSIN II RECEPTOR BLOCKER FOR PREVENTION OR TREATMENT OF NON-ALCOHOLIC FATTY LIVER DISEASE**

(30) Priority: 20.12.2022 KR 20220179807
(71) Applicant: THPharm Corp., Cheongju-si, Chungcheongbuk-do 28160 (KR)
(72) Inventor: HAN, Tae Hee, Cheongju-si, Chungcheongbuk-do 28165 (KR); YOO, Yung Geun, Seoul 06256 (KR)
(74) Representative: Cabinet Becker et Associés
(86) International application number: PCT/KR2023/095114
(87) International publication number: WO 2024/136627

(57) **Abstract**

The present disclosure relates to a pharmaceutical composition for the prevention or treatment of non-alcoholic fatty liver disease that contains a sodium-glucose transporter-2 inhibitor and an angiotensin II receptor blocker. With the absence of drug interaction problems and a synergistic effect in the prevention or treatment of non-alcoholic fatty liver disease, the pharmaceutical composition can be advantageously applied to patients with non-alcoholic fatty liver disease.

## Description

### [Technical Field]

The present disclosure relates to a pharmaceutical composition containing a sodium glucose transporter-2 inhibitor and an angiotensin II receptor blocker for the prevention or treatment of non-alcoholic fatty liver disease.

The present disclosure was made with the support of the National Research Foundation of "Local Government-University Cooperation-Based Regional Innovation Project (Chungbuk Regional Innovation Platform, Project Unique Number 1345370811, Project Number 2021RIS-001)" with the Ministry of Education's funding in 2023.

This research was supported by "Regional Innovation Strategy (RIS)" through the National Research Foundation of Korea(NRF) funded by the Ministry of Education(MOE)(2021RIS-001).

### [Background Art]

Non-alcoholic fatty liver disease is reported as a disease diagnosed when fat deposition is observed within hepatocytes through imaging or histology, and there is no liver disease caused by significant alcohol consumption, continuous drug use, and other causes including genetic diseases as the cause of fat accumulation within hepatocytes. In addition, this disease is known to encompass simple fatty liver, non-alcoholic steatohepatitis, and non-alcoholic fatty liver-associated cirrhosis.

The non-alcoholic fatty liver disease is a very common disease, and it is known that its prevalence is rapidly increasing along with an increase in occurrence of various metabolic syndromes such as obesity and diabetes, and the incidence of cirrhosis caused by non-alcoholic steatohepatitis or non-alcoholic steatohepatitis-associated liver fibrosis is higher than that of simple non-alcoholic fatty liver disease. In addition, it has also been reported to increase the risk of liver-related mortality as well as the incidence of liver failure or hepatocellular carcinoma due to cirrhosis.

The non-alcoholic fatty liver disease is reported to increase with age and to differ by sex, and is known to be caused by a decrease in insulin sensitivity and hormonal changes with age.

The non-alcoholic fatty liver disease is known to be closely related to metabolic syndrome, overweight and obesity, type 2 diabetes, dyslipidemia, and the like. It has been reported that 90% of patients with non-alcoholic fatty liver disease have at least one element of metabolic syndrome, and one-third thereof have metabolic syndrome. Overweight and obesity are risk factors for non-alcoholic fatty liver disease, but particularly, central obesity is known to be a strong risk factor.

Accordingly, the present inventors conducted research to develop a combination therapy that may be effectively applied to the treatment of non-alcoholic fatty liver disease, and completed the present disclosure.

### [Disclosure]

### [Technical Problem]

One object of the present disclosure is to provide a pharmaceutical composition for the prevention or treatment of non-alcoholic fatty liver disease, including an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

Another object of the present disclosure is to provide a method for preventing or treating non-alcoholic fatty liver disease, including administering the pharmaceutical composition to a subject.

Yet another object of the present disclosure is to provide a kit including the composition.

### [Technical Solution]

One aspect of the present disclosure provides a pharmaceutical composition for the prevention or treatment of non-alcoholic fatty liver disease, including an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

According to an embodiment of the present disclosure, the sodium-glucose cotransporter-2 (SGLT-2) inhibitor may be dapagliflozin or empagliflozin.

According to an embodiment of the present disclosure, the angiotensin II receptor blocker may be telmisartan.

According to an embodiment of the present disclosure, the non-alcoholic fatty liver disease may be simple steatosis or non-alcoholic steatohepatitis.

According to an embodiment of the present disclosure, the weight ratio of the angiotensin II receptor blocker and the sodium glucose transporter-2 inhibitor may be 2: 1 to 8 : 1.

### [Advantageous Effects]

According to the pharmaceutical composition for the prevention or treatment of non-alcoholic fatty liver disease containing the sodium glucose transporter-2 inhibitor and the angiotensin II receptor blocker, with the absence of drug interaction problems and a synergistic effect in the prevention or treatment of non-alcoholic fatty liver disease, the pharmaceutical composition can be advantageously applied to patients with non-alcoholic fatty liver disease.

### [Description of Drawings]

FIG. 1 is a graph showing a fat accumulation inhibition effect of dapagliflozin (DA) and/or telmisartan (Telm) for each treatment concentration.
FIG. 2A is a fluorescence micrograph showing normal HepG2 cells.
FIG. 2B is a fluorescence micrograph showing HepG2 cells induced into a non-alcoholic fatty liver condition by oleic acid (OA).
FIG. 2C is a fluorescence micrograph showing HepG2 cells induced into a non-alcoholic fatty liver condition by oleic acid and treated with 5 µM dapagliflozin and 10 µM telmisartan.
FIG. 3 is a graph showing an increase rate (fold change) of mRNA expression of PPAR-α compared to β-actin for each treatment concentration (5 µM + 5 µM, 5 µM + 10 µM, 10 µM + 20 µM) of dapagliflozin (DA) and telmisartan (Telm) in HepG2 cells induced to a non-alcoholic fatty liver condition by oleic acid (OA).
FIG. 4 is a graph showing an increase rate (fold change) of mRNA expression of PPAR-γ compared to β-actin for each treatment concentration (5 µM + 5 µM, 5 µM + 10 µM, 10 µM + 20 µM) of dapagliflozin (DA) and telmisartan (Telm) in HepG2 cells induced to a non-alcoholic fatty liver condition by oleic acid (OA).
FIG. 5 is a diagram showing a weight loss effect in male rats by nonclinical 13-week repeated administration of dapagliflozin and/or telmisartan.
FIG. 6 is a diagram showing a weight loss effect in female rats by nonclinical 13-week repeated administration of dapagliflozin and/or telmisartan.

### [Best Mode]

One aspect of the present disclosure provides a pharmaceutical composition for the prevention or treatment of non-alcoholic fatty liver disease, including an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

The SGLT-2 inhibitor included in the pharmaceutical composition of the present disclosure may be any one substance selected from the group consisting of dapagliflozin, empagliflozin, ipragliflozin, canagliflozin, luseogliflozin, and tofogliflozin, but preferably dapagliflozin or empagliflozin in terms of combination compatibility and synergistic effect, and most preferably dapagliflozin.

The dose of the pharmaceutical composition according to one embodiment of the present disclosure may be, for example, within the range of about 0.001 mg/kg to about 100 mg/kg, about 0.01 mg/kg to about 10 mg/kg, or about 0.1 mg/kg to about 1 mg/kg for adults, may be administered once a day, multiple times a day, or once a week, once every two weeks, once every three weeks, or once every four weeks to once a year, and may be administered once or several times a day at regular intervals according to the judgment of a doctor or pharmacist.

The pharmaceutical composition of the present disclosure may be prepared by mixing the angiotensin II receptor blocker as the first pharmaceutical ingredient and the SGLT-2 inhibitor as the second pharmaceutical ingredient, and the mixing thereof may be performed simultaneously or sequentially, and performed using methods known in the art.

The pharmaceutical composition of the present disclosure may be, for example, a double-layered tablet including the angiotensin II receptor blocker as the first pharmaceutical ingredient and the SGLT-2 inhibitor as the second pharmaceutical ingredient, or an inner core tablet structure including the angiotensin II receptor blocker as the first pharmaceutical ingredient and the SGLT-2 inhibitor as the second pharmaceutical ingredient and an outer layer surrounding an inner core layer, but is not limited thereto.

The composition of the present disclosure may include a pharmaceutically acceptable carrier. The pharmaceutically acceptable carrier included in the composition of the present disclosure is generally used in preparation of drugs, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition of the present disclosure may further include a lubricant, a wetting agent, a sweetening agent, a flavoring agent, an emulsion, a suspension, a preservative, and the like, in addition to the ingredients. Suitable pharmaceutically acceptable carriers and preparations are described in detail in Remington: the science and practice of pharmacy 22nd edition (2013).

According to one embodiment of the present disclosure, the pharmaceutical composition may be administered together with at least one substance that exhibits the pharmaceutical activity for non-alcoholic fatty liver disease.

In addition, the pharmaceutical composition according to one embodiment of the present disclosure may be used alone or in combination with methods using surgery, hormone therapy, drug therapy, and/or biological response regulators, for the prevention or treatment of non-alcoholic fatty liver disease.

The composition of the present disclosure may include various bases and/or additives necessary and appropriate for the preparation of formulations thereof, and without departing from the range of reducing the effects thereof, may be prepared by further including known compounds, such as nonionic surfactants, silicone polymers, extenders, fragrances, preservatives, disinfectants, oxidation stabilizers, organic solvents, ionic or nonionic thickeners, softeners, antioxidants, free radical destroyers, opacifiers, stabilizers, emollients, silicone, α-hydroxy acid, anti-foaming agents, moisturizers, vitamins, insect repellents, flavorings, preservatives, surfactants, anti-inflammatory agents, substance P antagonists, fillers, polymers, propellants, alkalinizing or acidifying agents, or coloring agents.

A suitable dose of the composition of the present disclosure may be variously prescribed by factors, such as a formulation method, an administration method, age, weight, sex, and a pathological condition of a patient, diet, an administration time, an administration route, an excretion rate, and response susceptibility. The dose of the composition of the present disclosure may be 0.001 to 1000 mg/kg for adults.

The composition of the present disclosure may be orally administered.

The composition of the present disclosure may be administered in various formulations when administered orally, and may be administered in the form of tablets, pills, hard/soft capsules, liquids, suspensions, emulsifiers, syrups, granules, elixirs, troches, etc., and may further include various excipients, such as wetting agents, sweeteners, aromatics, preservatives, etc. Specifically, when the composition of the present disclosure is prepared in the form of oral administration formulations, the composition may further include suitable carriers, excipients and diluents commonly used in preparation thereof. The carriers, excipients and diluents may be used with, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and/or mineral oil, but are not limited thereto. In addition, the composition may be prepared by including diluents or excipients, such as fillers, extenders, binders, wetting agents, disintegrants, and surfactants commonly used in preparation, and may further include lubricants such as magnesium stearate or talc, in addition to the excipients.

The composition of the present disclosure may be composed of about 0.1% to 90%, preferably about 1% to about 80% of a pharmaceutical ingredient. Pharmaceutical preparations for enteral or parenteral administration may be prepared by known methods, for example, by conventional mixing, granulating, coating, solubilizing or freeze-drying methods. Pharmaceutical preparations for oral use may be obtained by mixing the active ingredient with a solid excipient, preferably granulating the obtained mixture, and, if necessary or essential, processing the mixture or granules into tablets or coated tablets, after adding suitable auxiliary substances.

According to an embodiment of the present disclosure, the SGLT-2 inhibitor may be dapagliflozin or empagliflozin.

As used herein, the term "dapagliflozin" refers to (1S)-1,5-anhydro-1-C-{4-chloro-3-[(4-ethoxyphenyl)methyl]phenyl}-D-glucitol, and "empagliflozin" refers to (1S)-1,5-anhydro-1-C-[4-chloro-3-[[4-[[(3S)-tetrahydro-3-furanyl]oxy]phenyl]methyl]phenyl]-D-glucitol.

A preferred dose unit form of the SGLT-2 inhibitor may be, for example, a tablet or capsule containing about 5 mg to about 800 mg, preferably about 50 mg to about 700 mg, more preferably about 100 mg to about 600 mg, and most preferably about 100 mg to about 300 mg, which is administered once daily.

According to an embodiment of the present disclosure, the angiotensin II receptor blocker may be telmisartan.

As used herein, the term "telmisartan" refers to 4'-[(1,4'-dimethyl-2'-propyl[2,6'-bi-1H-benzimidazol]-1'-yl)methyl][1,1'-biphenyl]-2-carboxylic acid.

The telmisartan may be supplied in a suitable dose unit form, for example, in the form of capsules or tablets, containing a therapeutically effective amount, for example, about 20 to about 320 mg of telmisartan, which may be applied to a patient. The active ingredient may be administered up to three times daily, while increasing from a daily dose of, for example, 20 mg or 40 mg of telmisartan to a daily dose of 80 mg and then to a daily dose of 160 mg to 320 mg. For example, the active ingredient may be administered in the morning, midday or evening. For non-alcoholic fatty liver disease, once-daily or twice-daily administration is preferable.

The dapagliflozin, empagliflozin or telmisartan may be referred to including both active metabolites and prodrugs thereof. The "metabolite" is each active derivative that may be produced when dapagliflozin, empagliflozin or telmisartan is metabolized, and the "prodrug" refers to a compound that is metabolized to dapagliflozin, empagliflozin or telmisartan, or to the same metabolite(s) as dapagliflozin, empagliflozin or telmisartan. In addition, dapagliflozin, empagliflozin or telmisartan may include all pharmaceutically acceptable salts thereof, crystal forms thereof, hydrates, solvates, diastereomers or enantiomers.

The composition of the present disclosure may further contain vitamin D. A preferred dose unit form of vitamin D is, for example, tablets or capsules containing about 0.5 mg to about 1,200 mg, preferably about 5 mg to about 600 mg, and the daily dose is preferably administered once or twice daily.

According to an embodiment of the present disclosure, the non-alcoholic fatty liver disease may be simple steatosis or non-alcoholic steatohepatitis.

Recently, in major liver societies around the world, including the United States and Europe, 'non-alcoholic fatty liver disease (NAFLD)' is retitled to 'metabolic dysfunction-associated steatotic liver disease (MASLD),' and 'non-alcoholic steatohepatitis (NASH)' is retitled to 'metabolic dysfunction-associated steatohepatitis (MASH)'.

Accordingly, the term 'non-alcoholic fatty liver disease (NAFLD)' used herein is used with the same meaning as 'metabolic dysfunction-associated steatotic liver disease (MASLD)'.

In addition, the term 'non-alcoholic steatohepatitis (NASH)' used herein is used with the same meaning as 'metabolic dysfunction-associated steatohepatitis (MASH)'.

According to one embodiment of the present disclosure, the weight ratio of the angiotensin II receptor blocker and the sodium glucose transporter-2 inhibitor may be 2: 1 to 8: 1.

If the angiotensin II receptor blocker is included in less than 2 parts by weight or more than 8 parts by weight based on 1 part by weight of the sodium glucose transporter-2 inhibitor, an intracellular fat accumulation inhibition effect and a weight loss effect may not be sufficient, and thus the sufficient effect for preventing and treating non-alcoholic fatty liver disease may not be achieved.

Another aspect of the present disclosure provides a method for preventing or treating non-alcoholic fatty liver disease, including administering the pharmaceutical composition to a subject.

An angiotensin II receptor blocker or a pharmaceutically acceptable salt thereof, and an SGLT-2 inhibitor or a pharmaceutically acceptable salt thereof, included in the pharmaceutical composition of the present disclosure, may be administered orally in an amount effective for the treatment or prevention to a subject or patient, depending on the intended purpose. It should be understood that the dose for a specific subject or patient should be determined based on various related factors, such as the patient's weight, age, race, sex, health condition, diet, administration time, administration method, and severity of disease, and may be appropriately increased or decreased by a specialist. For example, a doctor may gradually increase the dose of the pharmaceutical composition of the present disclosure at a level lower than that required to achieve a desired therapeutic effect until the desired effect is achieved, and may easily determine and prescribe the dose as needed.

The method for preventing or treating metabolic disease of the present disclosure may be administering a therapeutically effective amount of each pharmaceutical ingredient of the composition simultaneously or sequentially or in any order.

The corresponding active ingredient or a pharmaceutically acceptable salt thereof may be used in the form of hydrates or may include other solvents used for crystallization. For example, 12.3 mg of a dapagliflozin propanediol hydrate is equivalent to 10 mg when converted to dapagliflozin as the pharmaceutical ingredient.

Yet another aspect of the present disclosure provides a kit including the composition.

The kit of the present disclosure may include an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient. The kit form may administer separate pharmaceutical ingredients in the same administration form or in different administration forms (e.g., the first pharmaceutical ingredient is parenterally administered and the second pharmaceutical ingredient is orally administered), and may be particularly advantageous when administered at multiple administration intervals.

### [Modes of the Invention]

Hereinafter, the present disclosure will be described in more detail with reference to Examples. However, these Examples are only illustrative the present disclosure, and the scope of the present disclosure is not limited to these Examples.

### Example 1. Confirmation of excellent fat accumulation inhibition effect of composite ingredient of dapagliflozin and telmisartan in non-alcoholic fatty liver induced cells

### 1-1. Induction of non-alcoholic fatty liver disease and treatment of experimental ingredient

HepG2 cells were treated with oleic acid (OA) to induce a non-alcoholic fatty liver disease condition, and a fat accumulation inhibition effect was evaluated in cells in which non-alcoholic fatty liver disease was induced by treating the experimental ingredient.

Specifically, HepG2 cells cultured under the conditions of 37°C and 5% CO₂ in a DMEM medium supplemented with 10% FBS and 1% penicillin/streptomycin were dispensed at the cell number of 1 × 10⁵ cells/well in a DMEM medium supplemented with 20mM% oleic acid in a 6-well plate to induce fat accumulation for 3 days. The DMEM medium supplemented with oleic acid was replaced at daily intervals. While inducing fat accumulation in HepG2 cells, dapagliflozin (DA) as a sodium-glucose cotransporter-2 (SGLT-2) inhibitor and/or telmisartan (Telm) as an angiotensin II receptor blocker (ARB) were treated to each culture medium for each concentration as shown in Table 1, and after 3 days, the degree of fat production was observed to evaluate the fat accumulation inhibition effects of a single ingredient and a composite ingredient at different concentrations. In Table 1, Group A used normal cells in which non-alcoholic fatty liver was not induced, and Groups B to I used cells in which non-alcoholic fatty liver was induced with oleic acid.

**[Table 1]**

| Classification | Experimental ingredient | |
|---|---|---|
| | SGLT-2 inhibitor | ARB |
| A | - | - |
| B | Dapagliflozin 5 µM | - |
| C | - | Telmisartan 5 µM |
| D | Dapagliflozin 10 µM | - |
| E | - | Telmisartan 10 µM |
| F | Dapagliflozin 5 µM | Telmisartan 5 µM |
| G | Dapagliflozin 5 µM | Telmisartan 10 µM |
| H | - | Telmisartan 20 µM |
| I | Dapagliflozin 10 µM | Telmisartan 20 µM |

### 1-2. Measurement of intracellular neutral fat content by Nile Red Assay/Staining method

In order to evaluate a fat accumulation inhibition effect in non-alcoholic fatty liver induced cells treated with the experimental ingredient in Example 1-1, the intracellular neutral fat content was measured using a modified Nile Red Assay/Staining method.

Specifically, a culture medium of the cells treated with the experimental ingredient in Example 1-1 was discarded, washed twice with PBS, and then 10 µl of 4% formaldehyde was added to each well and fixed at room temperature for 5 hours. Thereafter, formaldehyde was removed, washed twice with PBS, and then an AdipoRed assay reagent and PBS were mixed in a ratio of 50 : 1, and 100 µl of the mixture was added to each well, and stained for 10 minutes at room temperature. Fluorescence measurement was performed using a Multifunctional Micro-plate Reader at excitation 485 nm and emission 535 nm.

### 1-3. Measurement of mRNA expression levels of PPAR-α and PPAR-γ

In order to determine a molecular biological mechanism of the non-alcoholic fatty liver improvement effect in non-alcoholic fatty liver induced cells treated with the experimental ingredient in Example 1-1, the relative level of mRNA expression for genes related to intracellular fatty acid synthesis and oxidation was measured by RT-PCR.

Specifically, cells treated with the experimental ingredient in Example 1-1 were washed twice with PBS, and then total RNA was extracted using an RNA Mini kit. cDNA was synthesized using 1 µg of the extracted RNA, and real-time PCR was performed using SYBR Green and the PPAR-α and PPAR-γ primers in Table 2, and β-actin was used as a control gene.

**[Table 2]**

| Primer name | | Nucleic acid sequence (5'→3') | SEQ ID NO: |
|---|---|---|---|
| PPAR-alpha | forward | TCCTGAGCCATGCAGAATTTAC | SEQ ID NO: 1 |
| | reverse | AGTCTAAGGCCTCGCTGGTG | SEQ ID NO: 2 |
| PPAR-gamma | forward | ATTCCATTCACAAGAACAGATCCAG | SEQ ID NO: 3 |
| | reverse | TTTATCTCCACAGACACGACATTCA | SEQ ID NO: 4 |
| beta-actin | forward | CCTGGCACCCAGCACAAT | SEQ ID NO: 5 |
| | reverse | GCCGATCCACACACGGAGTACT | SEQ ID NO: 6 |

A PCR reagent composition was used at 8 µl by diluting cDNA to 1/100 with distilled water, and used by adjusting the total volume to 25 µl using 0.5 µl of each 10 pmole primer, 12.5 µl of ROX plus, and 4 µl of distilled water. Fluorescence signal quantification was measured using the MAX Pro program.

### 1-4. Confirmation of fat accumulation inhibition effect of composite ingredient of dapagliflozin and telmisartan

For non-alcoholic fatty liver induced cells treated with the experimental ingredient in Example 1-1, the intracellular neutral fat content was measured using the method of Example 1-2.

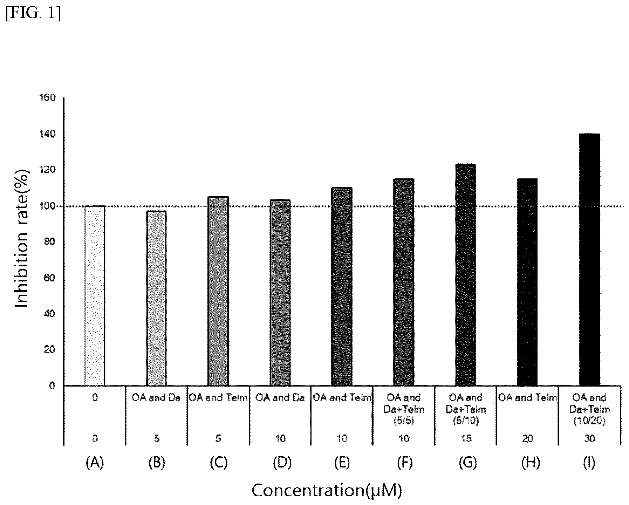

As a result of comparing a group treated with a single ingredient of dapagliflozin or telmisartan and a group treated with a composite ingredient, the group treated with the composite ingredient of dapagliflozin and telmisartan showed a relatively high inhibition rate of fat accumulation, and in particular, the composite ingredient of dapagliflozin and telmisartan showed the most effective inhibition rate in a concentration-dependent manner (FIG. 1).

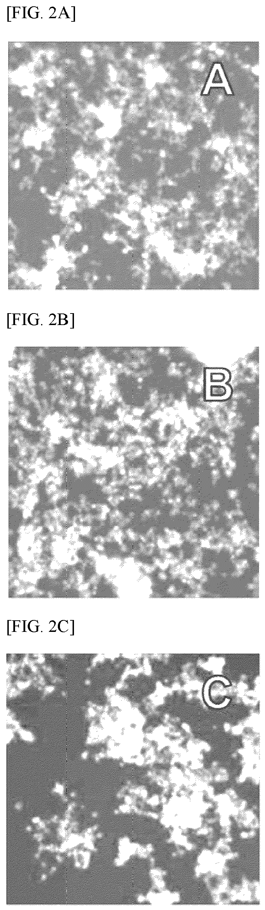

Meanwhile, in Group G treated with 5 µM dapagliflozin and 10 µM telmisartan, which showed a particularly remarkable synergistic effect compared to the group treated with the single ingredient, fat accumulation was observed using a fluorescence microscope, and as a result, it was confirmed that fat accumulation was effectively inhibited (FIGS. 2A, 2B and 2C).

Through these results, it was confirmed that the composite ingredient of dapagliflozin and telmisartan exhibited an excellent fat accumulation inhibition effect in HepG2 cells with fat accumulated by oleic acid.

### 1-5. Confirmation of fat accumulation inhibition mechanism according to regulation of PPAR-α and PPAR-γ mRNA expression by composite ingredient of dapagliflozin and telmisartan

For non-alcoholic fatty liver induced cells treated with the experimental ingredient in Example 1-1, the relative levels of mRNA expression for PPAR-α and PPAR-y, genes related to intracellular fatty acid synthesis and oxidation, were measured by RT-PCR using the method of Example 1-3.

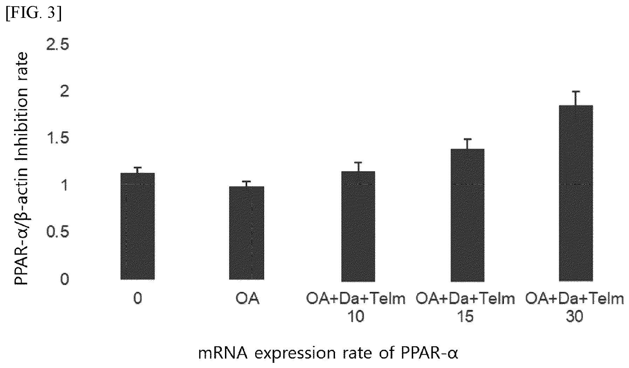

As a result, it was confirmed that in HepG2 cells treated with 20mM% oleic acid, the expression of PPAR-α mRNA was decreased compared to Group A that was not treated with oleic acid, and was significantly increased all in Groups F (5 µM and 5 µM), G (5 µM and 10 µM), and I (10 µM and 20 µM) treated with the composite ingredient of dapagliflozin and telmisartan (FIG. 3).

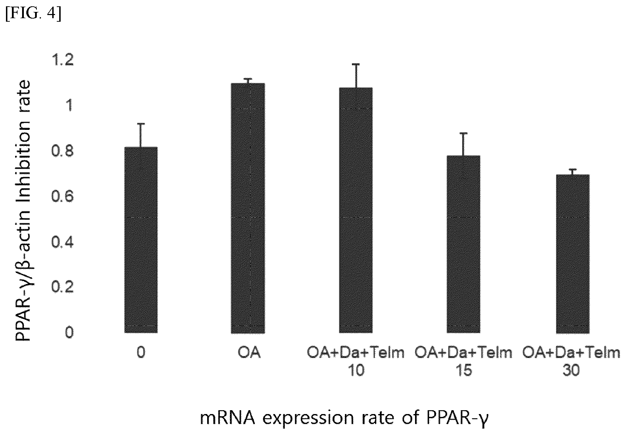

In addition, it was confirmed that in HepG2 cells treated with 20mM% oleic acid, the expression of PPAR-γ mRNA was increased compared to Group A that was not treated with oleic acid, and was decreased in a concentration-dependent manner all in Groups F (5 µM and 5 µM), G (5 µM and 10 µM), and I (10 µM and 20 µM) treated with the composite ingredient of dapagliflozin and telmisartan (FIG. 4).

Through these results, it was confirmed that in HepG2 cells with fat accumulated by oleic acid, the composite ingredient of dapagliflozin and telmisartan effectively inhibited fat accumulation by increasing the expression of PPAR-α to promote fat oxidation and rather decreasing the expression of PPAR-γ to effectively inhibit the fat accumulation.

### Example 2. Confirmation of excellent weight loss effect of composite ingredient of dapagliflozin and telmisartan through nonclinical 13-week repeated administration

### 2-1. Nonclinical 13-week repeated administration method

For the composite ingredient of dapagliflozin and telmisartan, which was confirmed to effectively inhibit fat accumulation in a non-alcoholic fatty liver cell model, the weight loss effect was confirmed through nonclinical 13-week repeated administration.

Specifically, groups were configured as shown in Table 3, and 6-week-old 10 male and 10 female Sprague-Dawley rats per group were orally administered repeatedly every day for 13 weeks, and during the test, general symptoms observation, blood tests, and body weight measurement were performed, and after the observation period was completed, the rats were euthanized and autopsied.

**[Table 3]**

| Group | | Dose (mg/kg/day) | | Dose volume (mL/kg) |
|---|---|---|---|---|
| | | Dapagliflozin | Telmisartan | |
| G1 | Control group | 0 | 0 | 10 |
| G2 | Composite low-dose group | 1 | 8 | 10 |
| G3 | Composite medium-dose group | 3 | 24 | 10 |
| G4 | Composite high-dose group | 9 | 72 | 10 |
| G5 | Dapagliflozin-alone administration group | 9 | 0 | 10 |
| G6 | Telmisartan-alone administration group | 0 | 72 | 10 |

Dapagliflozin was used with a dapagliflozin propanediol hydrate, and telmisartan was used with telmisartan powder (23.9%), and each was administered according to a dose.

### 2-2. Confirmation of weight loss effect of composite ingredient of dapagliflozin and telmisartan

Weight loss may reduce fat and inflammation in the liver, and alleviate conditions that contribute to fatty liver disease, which is one of the best treatments for non-alcoholic fatty liver disease (NAFLD) and non-alcoholic steatohepatitis (NASH). It has been reported that even a weight loss of 3 to 5% may reduce liver fat, and in the weight loss of 7% or more, inflammation is also reduced (The Korean Journal of Internal Medicine: Vol. 79, No. 5, 2010; Dig Dis Sci 52:589-593, 2007; J Hepatol In:103-107, 1997).

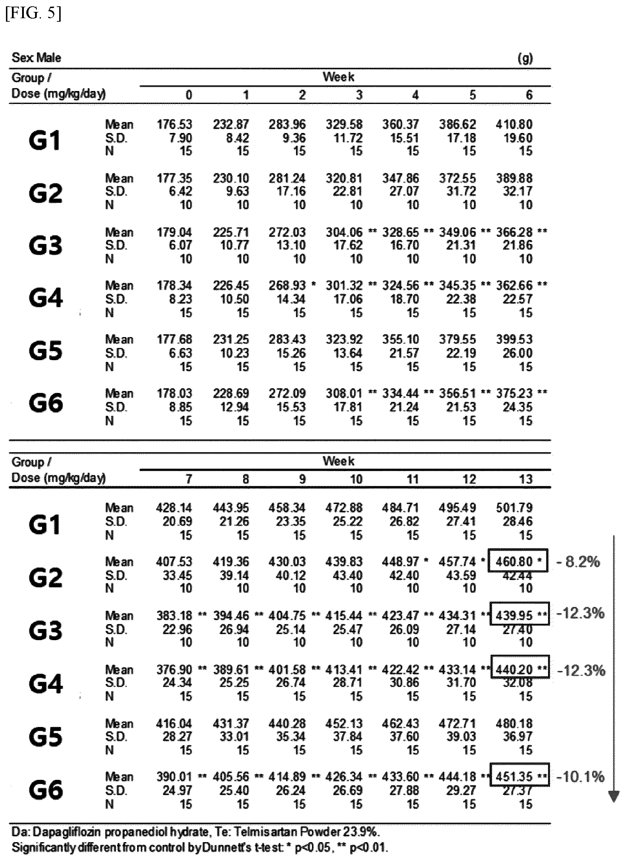

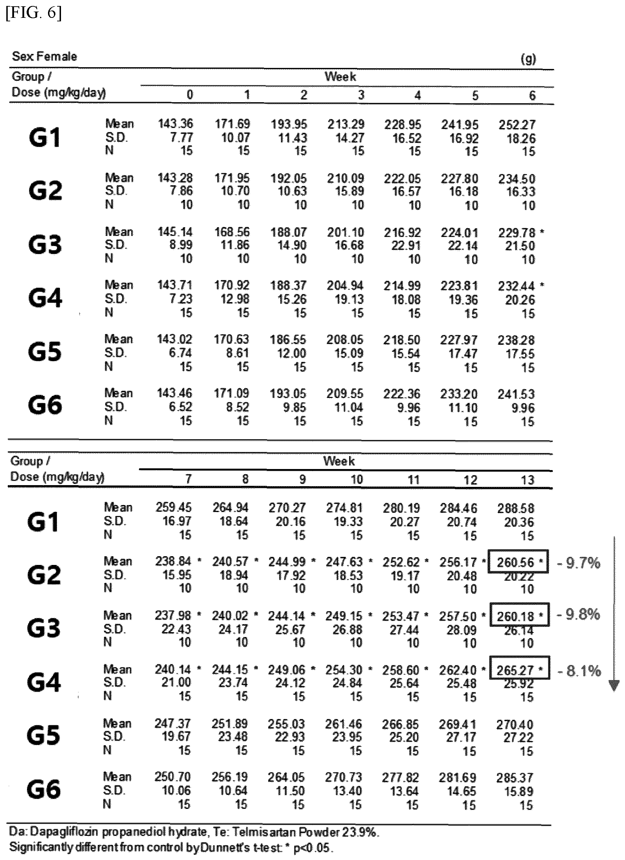

After repeated oral administration of dapagliflozin and/or telmisartan alone/composite ingredient for 13 weeks, weight loss effects were confirmed in the low, medium, and high-dose groups of composite ingredient of dapagliflozin and telmisartan. On the other hand, in the telmisartan-alone administration group, no weight loss effect was observed in females, and in the dapagliflozin-alone administration group, no weight loss effect was observed in either females or males (FIGS. 5 and 6).

Through these results, the synergistic effect of the composite ingredient of dapagliflozin and telmisartan on weight loss was confirmed, and it was confirmed that the therapeutic effect of non-alcoholic fatty liver disease may be achieved through the synergistic effect.

The present disclosure has been described above with reference to the embodiments thereof. It will be understood to those skilled in the art that the present disclosure may be implemented as modified forms without departing from an essential characteristic of the present disclosure. Therefore, the disclosed embodiments should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

## Claims

1. A pharmaceutical composition for the prevention or treatment of non-alcoholic fatty liver disease comprising:
an angiotensin II receptor blocker or a pharmaceutically acceptable salt thereof as a first pharmaceutical ingredient; and
a sodium-glucose cotransporter-2 (SGLT-2) inhibitor or a pharmaceutically acceptable salt thereof as a second pharmaceutical ingredient.

2. The pharmaceutical composition for the prevention or treatment of non-alcoholic fatty liver disease of claim 1, wherein the sodium-glucose cotransporter-2 (SGLT-2) inhibitor is dapagliflozin or empagliflozin.

3. The pharmaceutical composition for the prevention or treatment of non-alcoholic fatty liver disease of claim 1, wherein the angiotensin II receptor blocker is telmisartan.

4. The pharmaceutical composition for the prevention or treatment of non-alcoholic fatty liver disease of claim 1, wherein the non-alcoholic fatty liver disease is simple steatosis or non-alcoholic steatohepatitis.

5. The pharmaceutical composition for the prevention or treatment of non-alcoholic fatty liver disease of claim 1, wherein the weight ratio of the angiotensin II receptor blocker and the sodium glucose transporter-2 inhibitor is 2: 1 to 8 : 1.

6. A method for preventing or treating non-alcoholic fatty liver disease comprising:
administering to a subject a pharmaceutical composition comprising an angiotensin II receptor blocker or a pharmaceutically acceptable salt thereof as a first pharmaceutical ingredient; and
a sodium-glucose cotransporter-2 (SGLT-2) inhibitor or a pharmaceutically acceptable salt thereof as a second pharmaceutical ingredient.
